# EUROPEAN PATENT APPLICATION

(11) **EP 4 563 155 A1**
(43) Date of publication of application: **04.06.2025**
(21) Application number: 23860880.6
(22) Date of filing: 30.08.2023
(51) Int. Cl.: A61K 33/30, A61K 38/05, A61P 11/00, A23L 33/16, A23L 33/18

(54) **USE OF COMPOSITION CONTAINING ZINC SALT AND CYCLO-HISPRO FOR PREVENTION, ALLEVIATION OR TREATMENT OF RESPIRATORY DISEASE**

(30) Priority: 31.08.2022 KR 20220110114
(71) Applicant: Novmetapharma Co., Ltd., Seoul 06050 (KR)
(72) Inventor: JUNG, Hoe Yune, Pohang-si, Gyeongsangbuk-do 37668 (KR); JEON, Jong Su, Pohang-si, Gyeongsangbuk-do 37664 (KR); LEE, Do Hyun, Pohang-si, Gyeongsangbuk-do 37669 (KR); PARK, Onyu, Pohang-si, Gyeongsangbuk-do 37668 (KR); BAEK, Seo Yeong, Pohang-si, Gyeongsangbuk-do 37694 (KR)
(74) Representative: Maiwald GmbH
(86) International application number: PCT/KR2023/012909
(87) International publication number: WO 2024/049204

(57) **Abstract**

The present invention relates to the use of a composition containing a zinc salt and cyclo-hispro (CHP) for the prevention, alleviation, or treatment of respiratory diseases and, more specifically, provides a composition for prevention, alleviation, or treatment of respiratory diseases and a method for preventing, alleviating, or treating respiratory diseases by using same, wherein the composition includes a specific weight ratio of a zinc salt and cyclo-hispro as active ingredients, thereby exhibiting excellent effects of reducing allergic cytokines and decreasing mucus secretion.

## Description

### Technical Field

The present application claims priority to Korean Patent Application No. 10-2022-0110114, filed on August 31, 2022, and all contents of the application disclosed in the specification and drawings are incorporated herein by reference.

The present invention relates to the use of a composition containing a zinc salt and cyclo-hispro (CHP) for preventing, alleviating, or treating respiratory diseases, and more specifically, the present invention provides a composition for the prevention, alleviation, or treatment of respiratory diseases and a method for preventing, alleviating, or treating respiratory diseases using the same, wherein the composition includes a specific weight ratio of a zinc salt and CHP as active ingredients, thereby exhibiting excellent effects of reducing allergic cytokines and decreasing mucus secretion.

### Background Art

Respiratory diseases are diseases related to the lungs and airways and may be primarily caused by weakened immunity, inflammation, bacterial or viral infection, inhalation of harmful particles due to, for example, fine dust or smoking, and aging. Typical respiratory diseases include pneumonia, rhinitis, asthma, bronchitis, tuberculosis, and chronic obstructive pulmonary disease (COPD).

Among them, asthma is a disease characterized by the hyper-responsiveness of the airway to various stimuli, and symptoms such as wheezing, dyspnea, and coughing are exhibited due to extensive narrowing of the airway. Most cases of asthma are allergy-induced, and symptoms appear when the airway is exposed to external causative antigens. The most common causative antigen is house dust mite (HDM), but other causative antigens include pollen, mold, cockroaches, and animal hair. In general, it is common to test positive in skin or bronchial provocation tests for causative antigens, and the age of onset is relatively young.

Asthma is also recognized as a chronic inflammatory disease because inflammatory cells proliferate, differentiate, and become activated by IL-4, IL-5, and IL-13 produced by TH2 type immune cells and gather in the airways and the surrounding tissues. It is known that inflammatory cells such as eosinophils, mast cells, and alveolar macrophages activated in the bronchi secrete various inflammatory mediators and thus are involved in strong bronchoconstriction. Therefore, the production of cytokines such as IL-4, IL-5, and IL-13 and Ig-E serves as a major cause of inflammation and allergic reactions as well as asthma caused thereby, and therefore much research is being conducted on the development of drugs to suppress them.

Currently, steroids, bronchodilators, and antibiotics are mainly used for asthma treatment. Steroids and antibiotics are used to treat asthma by suppressing immune responses and inflammatory responses, while bronchodilators are used to offset clinical symptoms such as dyspnea when they occur. Inhaled corticosteroids, which are the most extensively used at present, show excellent therapeutic effects, but when used for a long time, they are known to cause adrenal suppression, decreased bone density, growth retardation, eye and skin complications, and increased collagen synthesis in proportion to the dose and duration of use. In addition, long-acting beta-2 agonists such as salmeterol and formeterol show a preventive effect on asthma attacks, but it has been reported that they may cause death in patients in some cases. In addition, side effects have been found with long-term use, limiting their use as asthma therapeutic agents. Therefore, there is a need for developing therapeutic agents with new mechanisms of treatment and fewer side effects.

Against this backdrop, the present inventors confirmed that a combination of zinc and cyclo-hispro (CHP) at a specific weight ratio exhibited a synergistic effect on reducing allergic cytokines and decreasing mucus secretion and thus showed an excellent respiratory disease treatment effect, thereby completing the present invention.

### [Related Art Documents]

### [Patent Documents]

(Patent Document D1) Korea Patent Publication No. 10-2022-0078140
(Patent Document D2) Korea Patent Publication No. 10-2264825

### Disclosure

### Technical Problem

Therefore, an object of the present invention is to provide a composition for preventing, alleviating, or treating respiratory diseases, including a combination of zinc and cyclo-hispro (CHP).

Another object of the present invention is to provide a method for preventing, alleviating, or treating respiratory diseases, using a combination of zinc and CHP.

Still another object of the present invention is to provide a use of a composition including a combination of zinc and CHP for the manufacture of a medicament for preventing, alleviating, or treating respiratory diseases.

### Technical Solution

To solve the above-described technical problems, the present invention provides a pharmaceutical composition for preventing or treating a respiratory disease, including: a zinc salt including a zinc cation and an anion; and CHP or a pharmaceutically acceptable salt thereof.

In addition, the present invention provides a health functional food composition for preventing or alleviating a respiratory disease, including: a zinc salt including a zinc cation and an anion; and CHP or a sitologically acceptable salt thereof.

In addition, the present invention provides a method for preventing, alleviating, or treating a respiratory disease, including administering an effective amount of a composition including: a zinc salt including a zinc cation and an anion; and CHP or a pharmaceutically acceptable salt thereof, to an individual in need thereof.

Furthermore, the present invention provides a use of a composition including a zinc salt including a zinc cation and an anion; and CHP or a pharmaceutically acceptable salt thereof, for the manufacture of a medicament for preventing, alleviating, or treating a respiratory disease.

In the present invention, the weight ratio of the zinc cation:CHP or a pharmaceutically or sitologically acceptable salt thereof may be 1:11 to 13.

In the present invention, the respiratory disease may be one or more diseases selected from the group consisting of respiratory inflammatory lung disease, chronic obstructive pulmonary disease (COPD), sinusitis, allergic rhinitis, lower respiratory tract infections, bronchitis, emphysema, pneumonia, asthma, bronchiectasis, emphysema, sequelae of pulmonary tuberculosis, acute respiratory distress syndrome (ARDS), cystic fibrosis, otitis media, and pulmonary fibrosis.

In the present invention, the bronchitis may be one or more selected from the group consisting of acute bronchitis, chronic bronchitis, allergic bronchitis, infectious bronchitis, traumatic bronchitis, catarrhal bronchitis, purulent bronchitis, obstructive bronchitis, ulcerative bronchitis, and infiltrative bronchitis.

In the present invention, the asthma may be one or more selected from the group consisting of bronchial asthma, atopic asthma, non-atopic asthma, exercise-induced asthma, aspirin-induced asthma, psychogenic asthma, and alveolar asthma.

In the present invention, the composition may exhibit the effects of i) reducing allergic cytokines and ii) decreasing mucus secretion.

In the present invention, the zinc salt and cyclo-hispro or a pharmaceutically or sitologically acceptable salt thereof are administered simultaneously, separately, or sequentially.

### Advantageous Effects

The composition including a zinc salt and cyclo-hispro at a specific weight ratio according to the present invention exhibits a synergistic effect on reducing allergic cytokines and decreasing mucus secretion, and thus can be used as a therapeutic agent for various respiratory diseases including asthma and bronchitis. In addition, the composition of the present invention exhibits an effect upon oral administration, and thus can be utilized as a health functional food.

### Description of Drawings

FIG. 1 shows the results of measuring the changes in expression of allergic cytokine IL-4 according to the weight ratio of zinc (Zn) and cyclo-hispro (CHP) in lung tissue with house dust mite (HDM)-induced inflammation.
FIG. 2 shows the results of measuring the changes in expression of allergic cytokine IL-13 according to the weight ratio of Zn and CHP in lung tissue with HDM-induced inflammation.
FIG. 3 shows the results of measuring the changes in expression of allergic cytokine IL-17a according to the weight ratio of Zn and CHP in lung tissue with HDM-induced inflammation.
FIG. 4 shows the results of measuring the changes in expression of mucin gene muc5ac according to the weight ratio of Zn and CHP in lung tissue with HDM-induced inflammation.

### Best Mode

As described above, various side effects of existing asthma therapeutic agents have been reported, and therefore, there is still a need for developing a therapeutic agent with a new mechanism and fewer side effects. Accordingly, the present inventors sought a solution to the above-described problem by experimentally verifying that a combination of zinc and cyclo-hispro (CHP) at a specific weight ratio exhibits a synergistic effect on reducing allergic cytokines and decreasing mucus secretion.

Therefore, the present invention relates to a composition for preventing, alleviating, or treating a respiratory disease, including a combination of zinc and CHP or a pharmaceutically acceptable salt thereof.

In the composition of the present invention, the zinc salt includes a zinc cation and an anion. At this time, as the anion, chloride, sulfate, or gluconate may be used, but it is not limited thereto.

In the present invention, "CHP" refers to a naturally occurring cyclic dipeptide consisting of histidine-proline, a metabolite of thyrotropin-releasing hormone (TRH), or a physiologically active dipeptide that is synthesized in the body through TRH metabolism *de novo,* and is a substance widely distributed throughout the brain, spinal cord, and gastrointestinal tract.

In the composition of the present invention, the CHP may be synthesized and used or commercially available CHP may be used. In addition, a substance containing CHP such as a prostate extract or soybean hydrolysate may be purified and used.

The term "purified" used herein means that the CHP is in a concentrated form compared to a form that may be obtained from natural sources such as a prostate extract. The purified components may be concentrated from their natural sources or obtained through chemical synthesis methods.

In the present invention, the zinc salt and CHP are also referred to as "Cyclo-Z", and the zinc salt and CHP may be included in the composition of the present invention in the form of a complex or as individual components.

Therefore, the zinc salt and CHP may be administered in the form of a complex or may be administered simultaneously, separately, or sequentially as individual components.

In the composition of the present invention, the weight ratio of the zinc cation:CHP may be 1:11 to 13.

In a specific embodiment of the present invention, an asthma animal model produced by inducing asthma by intranasally administering house dust mite (HDM) was administered the zinc cation and CHP at different weight ratios of 2:1 (Experimental Group 1) and 1:11.9 (Experimental Group 2), and then the changes in expression of allergic cytokine genes (IL-4, IL-13, and IL-17a) and mucin gene (muc5ac) according to the weight ratio of the zinc cation and CHP were observed.

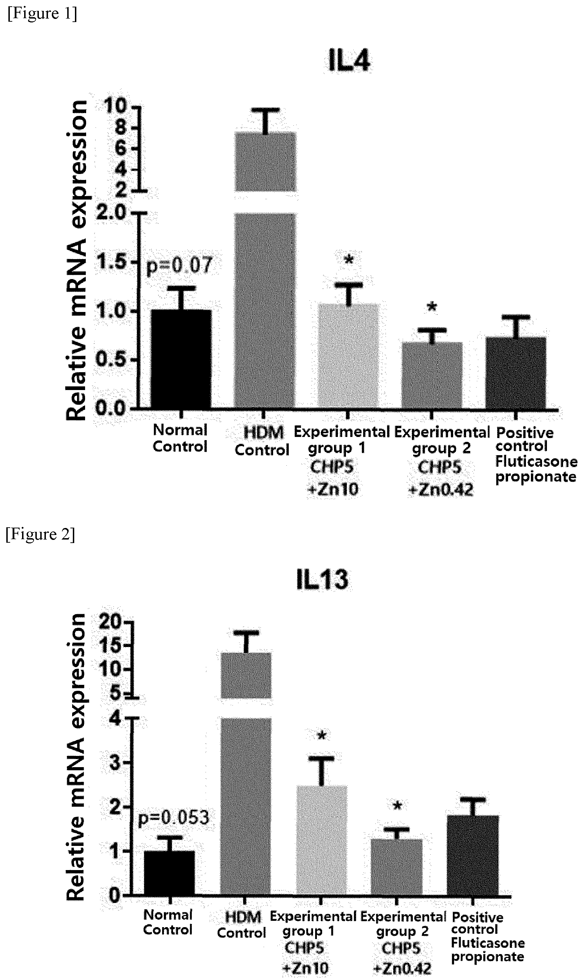

As a result, as confirmed in FIGS. 1 to 4, in both Experimental Groups 1 and 2 administered a combination of the zinc cation and CHP, the expression of allergic cytokines (IL-4, IL-13, and IL-17a) and mucin gene (muc5ac) increased by HDM were significantly reduced. In particular, Experimental Group 2 showed a significant reduction in allergic cytokine gene expression and mucin gene expression compared to not only Experimental Group 1 but also the positive control group (fluticasone propionate), despite maintaining the content of CHP and significantly decreasing the content of the zinc cation to 1/23 to 1/24 compared to Experimental Group 1. These results indicate that the combination of zinc cations and CHP showed the best synergistic effect.

Therefore, in the composition of the present invention, the weight ratio of the zinc cation:CHP is preferably 1:11 to 13, and when the zinc cation and CHP are administered at a weight ratio outside the above range, the intended synergistic effect may be reduced.

In the present invention, the term "synergistic effect" means that the effect that occurs when individual components are administered in combination is greater than the sum of the effects that occur when each component is administered alone as a single component [Chou and Talalay, Adv. Enzyme. Regul., 22:27-55, 1984]. The zinc salt and CHP or a pharmaceutically acceptable salt thereof of the present invention provide a synergistic effect of preventing, alleviating, or treating a bone loss disease by being administered in combination.

The term "administered in combination" means that the compounds or components are administered together to a patient. The fact that individual compounds or components are administered together means that the individual components may be sequentially administered at the same time or in any order or at different times in order to obtain the desired therapeutic effect.

In the present invention, the respiratory disease may be one or more diseases selected from the group consisting of respiratory inflammatory lung disease, chronic obstructive pulmonary disease (COPD), sinusitis, allergic rhinitis, lower respiratory tract infections, bronchitis, emphysema, pneumonia, asthma, bronchiectasis, emphysema, sequelae of pulmonary tuberculosis, acute respiratory distress syndrome (ARDS), cystic fibrosis, otitis media, and pulmonary fibrosis, but is not limited thereto.

In the present invention, the bronchitis may be one or more selected from the group consisting of acute bronchitis, chronic bronchitis, allergic bronchitis, infectious bronchitis, traumatic bronchitis, catarrhal bronchitis, purulent bronchitis, obstructive bronchitis, ulcerative bronchitis, and infiltrative bronchitis, but is not limited thereto.

In the present invention, the asthma may be one or more selected from the group consisting of bronchial asthma, atopic asthma, non-atopic asthma, exercise-induced asthma, aspirin-induced asthma, psychogenic asthma, and alveolar asthma, but is not limited thereto.

The composition of the present invention is effective in treating a respiratory disease including asthma and bronchitis because it reduces allergic cytokines (IL-4, IL-13, and IL-17a) and mucus secretion in lung tissue that are increased by external causative antigens such as HDM.

The term "prevention" used in the present invention refers to any act of suppressing or delaying the onset of a disease or condition. In the present invention, it means delaying the onset of a respiratory disease or suppressing the onset.

The term "alleviation" used in the present invention refers to any act of ameliorating or beneficially changing the state of a disease or condition, and in the present invention, it means ameliorating the symptoms of a respiratory disease.

The term "treatment" used in the present invention refers to any act of delaying, stopping, or reversing the progression of a disease or condition, and in the present invention, it means relieving, mitigating or eliminating, or reversing the symptoms of a respiratory disease.

The term "pharmaceutically acceptable salt" used in the present invention refers to any organic or inorganic addition salt of CHP, which has an effect at a concentration that is relatively non-toxic and harmless to the patient, and the side effects caused by this salt do not reduce the beneficial efficacy of CHP. Inorganic acids and organic acids may be used as free acids of these salts, and hydrochloric acid, hydrobromic acid, nitric acid, sulfuric acid, perchloric acid, phosphoric acid, and the like may be used as the inorganic acids, and citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D-) or (L-) malic acid, maleic acid, methanesulfonic acid, ethanesulfonic acid, 4-toluenesulfonic acid, salicylic acid, citric acid, benzoic acid, malonic acid, and the like may be used as the organic acids. In addition, these salts include alkali metal salts (sodium salts, potassium salts, etc.) and alkaline earth metal salts (calcium salts, magnesium salts, etc.). For example, acid addition salts may include acetate, aspartate, benzate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methyl sulfate, naphthylate, 2-naphthylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, and zinc salts.

The pharmaceutical composition of the present invention may further include a pharmaceutically acceptable carrier. The pharmaceutical composition including a pharmaceutically acceptable carrier may have various oral or parenteral dosage forms. When formulated, it may be prepared using diluents or excipients such as commonly used fillers, bulking agents, binders, wetting agents, disintegrants, and surfactants. Solid preparations for oral administration may include tablets, pills, powder, granules, capsules, troches, and the like, and such solid preparations may be prepared by mixing one or more compounds of the present invention with at least one excipient, for example, starch, calcium carbonate, sucrose, lactose, or gelatin. In addition to simple excipients, lubricants such as magnesium stearate and talc may also be used. Liquid preparations for oral administration may include suspensions, solutions, emulsions, or syrups. In addition to commonly used simple diluents such as water and liquid paraffin, various excipients such as wetting agents, sweeteners, flavoring agents, and preservatives may be included.

Preparations for parenteral administration may include sterilized aqueous solutions, non-aqueous solutions, suspensions, emulsions, lyophilized preparations, suppositories, and the like. As a non-aqueous solvent and a suspension, propylene glycol, polyethylene glycol, vegetable oils such as olive oil, and injectable esters such as ethyl oleate may be used. As a base for suppositories, Witepsol, Macrogol, Tween 61, cacao oil, laurel oil, glycerol, gelatin, or the like may be used.

The pharmaceutical composition of the present invention may be administered via any common route capable of reaching the target tissue or cell in an individual or a sample. The administration may include systemic or local administration, and may include intraperitoneal administration, intravenous administration, intramuscular administration, subcutaneous administration, intradermal administration, oral administration, intranasal administration, intrapulmonary administration, and rectal administration, but is not limited thereto. The dose of the pharmaceutical composition may vary depending on the patient's age, body weight, and sex, dosage form, health conditions, and disease severity.

The term "patient" refers to any single individual requiring treatment, including humans, cows, dogs, guinea pigs, rabbits, chickens, insects, and the like. Any subjects participating in a clinical trial who do not exhibit any clinical manifestations of disease, or subjects participating in an epidemiological study, or subjects used as controls are also included in the subjects.

The pharmaceutical composition of the present invention may provide a desirable effect of preventing, alleviating, or treating a respiratory disease when it contains an effective amount of a zinc salt and CHP or a pharmaceutically acceptable salt thereof. The term "effective amount" as used herein refers to an amount that exhibits a greater response than a negative control, and preferably refers to an amount sufficient to prevent, alleviate, or treat a respiratory disease. The pharmaceutical composition of the present invention may contain 0.01% to 99.9% of a zinc salt and CHP or a pharmaceutically acceptable salt thereof, and the remainder may be a pharmaceutically acceptable carrier. The effective amount of the zinc salt and CHP or a pharmaceutically acceptable salt thereof contained in the pharmaceutical composition of the present invention will vary depending on the form in which the composition is commercialized, and the like.

The total effective amount of the pharmaceutical composition of the present invention may be administered to a patient as a single dose, or may be administered by a fractionated treatment protocol in which multiple doses are administered over a long period of time. The content of the effective ingredient in the pharmaceutical composition of the present invention may vary depending on the severity of the disease. For example, the composition may be administered in one to several divided doses, preferably in an amount of 0.001 to 100 mg, more preferably 0.01 to 10 mg per kg of body weight per day based on the zinc salt and CHP or a pharmaceutically acceptable salt thereof. However, the dosage of the zinc salt and CHP or a pharmaceutically acceptable salt thereof is determined as an effective dosage for a patient by considering various factors such as the administration route of the pharmaceutical composition and the number of treatments as well as the patient's age, weight, health condition, and sex, disease severity, diet, and excretion rate. Therefore, by considering the above factors, a person having ordinary skill in the art will be able to determine an appropriate effective dosage of the zinc salt and CHP or a pharmaceutically acceptable salt thereof for a specific use for preventing, treating, or alleviating a respiratory disease. The formulation, administration route, and administration method of the pharmaceutical composition according to the present invention are not particularly limited as long as the effects of the present invention are exhibited.

In addition, the present invention relates to a health functional food composition for preventing or alleviating a respiratory disease, including: a zinc salt including a zinc cation and an anion; and CHP or a sitologically acceptable salt thereof.

In the health functional food composition of the present invention, the description of the composition and effect of the zinc salt and CHP included as effective ingredients are the same as described above, so their description is omitted.

In the present invention, the term "sitologically acceptable salt" includes a salt derived from a sitologically acceptable organic acid, inorganic acid, or base.

In the present invention, the term "health functional food" includes the meanings of both "functional food" and "health food."

In the present invention, the term "health functional food" is the same term as food for special health use (FoSHU) and refers to food with a high medicinal and healthcare effect that is processed to efficiently exhibit bioregulatory functions in addition to supplying nutrients.

In the present invention, the term "health food" refers to food that has a more active health maintenance or promotion effect than general food, and health supplement food refers to food for the purpose of health supplementation. In some cases, the terms health functional food, health food, and health supplement food may be used interchangeably. The food may be manufactured in various forms such as tablets, capsules, powder, granules, liquid, and pills to obtain useful effects in preventing or alleviating a respiratory disease.

As a specific example of such functional food, the zinc salt and CHP or a sitologically acceptable salt thereof of the present invention may be used to manufacture a processed food that changes the characteristics of agricultural products, livestock products, or fishery products while improving their storage properties.

The health functional food composition of the present invention may also be manufactured in the form of nutritional supplements, food additives, feed, and the like, and is intended for consumption by humans or animals including livestock.

The above types of food composition may be manufactured in various forms according to conventional methods known in the art. General foods may be manufactured by adding the zinc salt and CHP or a sitologically acceptable salt thereof of the present invention to beverages (including alcoholic beverages), fruits and processed foods thereof (e.g., canned fruits, bottled fruits, jams, marmalades, etc.), fish, meats, and processed foods thereof (e.g., ham, sausages, corned beef, etc.), breads and noodles (e.g., udon, buckwheat noodles, ramen, spaghetti, macaroni, etc.), fruit juices, various drinks, cookies, taffies, dairy products (e.g., butter, cheese, etc.), edible vegetable oils, margarine, vegetable proteins, retort food, frozen food, various seasonings (e.g., soybean paste, soy sauce, sauces, etc.), and the like, but are not limited thereto.

In addition, the nutritional supplement may be manufactured by adding the zinc salt and CHP or a sitologically acceptable salt thereof of the present invention to capsules, tablets, pills, and the like, but is not limited thereto.

In addition, as the health functional food, for example, the zinc salt and CHP or a sitologically acceptable salt thereof of the present invention may be manufactured in the form of tea, juice, and drink to be consumed (health drinks) by liquefying, granulating, encapsulating and powdering, but it is not limited thereto. In addition, in order to use the zinc salt and CHP or a sitologically acceptable salt thereof of the present invention in the form of a food additive, it may be manufactured in the form of powder or a concentrate and used. In addition, the zinc salt and CHP or a sitologically acceptable salt thereof of the present invention may be manufactured in the form of a composition by mixing with a known active ingredient known to be effective in preventing or alleviating a respiratory disease.

When the food composition of the present invention is used as a health beverage composition, the health beverage composition may contain various flavoring agents or natural carbohydrates as additional ingredients, like a typical beverage. The above-mentioned natural carbohydrates may include monosaccharides such as glucose and fructose; disaccharides such as maltose and sucrose; polysaccharides such as dextrin and cyclodextrin; and sugar alcohols such as xylitol, sorbitol, and erythritol. Natural sweeteners such as thaumatin and stevia extract, synthetic sweeteners such as saccharin and aspartame, and the like may be used as the sweeteners. The proportion of the natural carbohydrates is generally about 0.01 to 0.04 g, preferably about 0.02 to 0.03 g, per 100 mL of the composition of the present invention.

The zinc salt and CHP or a sitologically acceptable salt thereof of the present invention may be contained as an effective ingredient of a health functional food composition for preventing or alleviating a respiratory disease, and the amount thereof is an effective amount for obtaining the above-mentioned preventing or alleviating effect, for example, preferably 0.01% to 100% by weight based on the total weight of the entire composition, but is not particularly limited thereto. The health functional food composition of the present invention may be prepared in the form of a composition by mixing the zinc salt and CHP or a sitologically acceptable salt thereof with a known active ingredient that is known to be effective in preventing or alleviating a respiratory disease.

In addition, the health functional food of the present invention may contain various nutrients, vitamins, electrolytes, flavoring agents, coloring agents, pectic acid, salts of pectic acid, alginic acid, salts of alginic acid, organic acids, protective colloid thickeners, pH regulators, stabilizers, preservatives, glycerin, alcohols, or carbonating agents. In addition, the health functional food of the present invention may contain fruit pulp for the manufacture of natural fruit juice, fruit juice drinks, or vegetable drinks. These ingredients may be used independently or in combination. The proportion of these additives is not particularly important, but is generally selected in the range of 0.01 to 0.1 parts by weight per 100 parts by weight of the composition of the present invention.

Additionally, the present invention relates to a method for preventing, alleviating, or treating a respiratory disease, including administering an effective amount of a composition including: a zinc salt including a zinc cation and an anion; and CHP or a pharmaceutically acceptable salt thereof, to an individual in need thereof.

In the method of the present invention, the term "individual" includes, any animal (e.g., humans, horses, pigs, rabbits, dogs, sheep, goats, non-human primates, cows, cats, guinea pigs, or rodents), but is not limited thereto. This term does not indicate a particular age or sex. Therefore, it is intended to include female, male, adult, and newborn subjects as well as fetuses. A patient refers to a subject suffering from a disease or disorder. The term patient includes human and veterinary subjects.

In the method of the present invention, the description of the composition including the effect, administration route, number of administrations, dosage, and the like of the zinc salt and CHP or a pharmaceutically acceptable salt thereof administered to the individual is the same as described above, so their description is omitted.

In the method of the present invention, the zinc salt and CHP may provide a desirable effect of preventing, alleviating, or treating a respiratory disease when administered in an effective amount. For a desirable effect, the combination of the zinc salt and CHP or a pharmaceutically acceptable salt thereof of the present invention may be administered once or repeatedly several times at regular time intervals. At this time, the zinc salt and the CHP may be administered simultaneously, separately, or sequentially. For example, the zinc salt and CHP or a pharmaceutically acceptable salt thereof may be co-formulated and administered simultaneously as a combined unit dosage formulation, or may be administered simultaneously or sequentially as separate formulations.

When administered sequentially, individual active ingredients may be administered as individual formulations with time intervals, and the order of administration may be determined by a physician or a person of ordinary skill in the art.

In addition, it may be used in combination with other methods for preventing, alleviating, or treating a respiratory disease.

Furthermore, the present invention relates to a use of a composition including a zinc salt including a zinc cation and an anion; and CHP or a pharmaceutically acceptable salt thereof, for the manufacture of a medicament for preventing, alleviating, or treating a respiratory disease.

In the use of the present invention, the description of the composition including the effect, administration route, number of administrations, dosage, and the like of the zinc salt and CHP or a pharmaceutically acceptable salt thereof administered to the individual is the same as described above, so their description is omitted.

Hereinafter, the present invention will be described in more detail through examples. These examples are only for the purpose of illustrating the present invention, and it will be obvious to those skilled in the art that the scope of the present invention is not to be construed as being limited by these examples. It should be understood that the scope of the present invention includes all modifications, equivalents, and substitutes included in the spirit and technical scope of the present invention.

### Modes for Invention

### [Example 1]

### Establishment of an asthma animal model using HDM

### 1-1. Breeding of experimental animals and induction of an asthma animal model

An HDM (*D. pteronyssius*) extract for inducing an asthma animal model was purchased from GREER, and 6-week-old female Balb/c mice were purchased from KOATECH Co., Ltd.

After arrival, the Balb/c mice were housed in an environment of 23±3 °C with free access to food and water. After acclimating to the housing environment for one week and measuring body weight, they were divided into five groups as shown in Table 1 by randomly distributing them to ensure uniform average body weights. For HDM sensitization, 25 µg HDM per mouse was intranasally (i.n.) administered to all groups except the normal control on days 0 and 3. At this time, HDM was dissolved in 50 µl of saline before use. For subsequent challenges, the same amount of HDM was intranasally administered on days 10, 11, and 12. The normal control was injected with saline using the same method.

### 1-2. Administration of test substances

As shown in Table 1, immediately after the HDM stimulation on days 10, 11, and 12, Experimental Group 1 was orally administered 10 mg/kg Zn (zinc cation component) + 5 mg/kg CHP, and Experimental Group 2 was administered 0.42 mg/kg Zn (zinc cation component) + 5 mg/kg CHP dissolved in 200 µl of distilled water (DW). The normal control group and HDM control group were orally administered the same amount of DW. Fluticasone propionate used for the positive control was purchased from Angene Chemical. Fluticasone propionate was intranasally administered to the positive control at 20 µg per mouse. At this time, fluticasone propionate was first dissolved in 100% ethanol (pure grade) at a concentration of 20 mg/ml, and then dissolved in saline to reach a concentration of 20 µg/50 µl before use.

**[Table 1]**

| Group | Normal control (Vehicle) | HDM control (HDM) | Experimental Group 1 (Zn+CHP 15 mg/kg) | Experimental Group 2 (Zn+CHP 5.42 mg/kg) | Positive control (Fluticasone propionate) |
|---|---|---|---|---|---|
| HDM (i.n.) | Saline | HDM 25µg/mouse | | | |
| Administration | DW | DW | Zn 10 mg/kg | Zn 0.42 mg/kg | 20 µg/head |
| | | | CHP 5mg/kg | CHP 5 mg/kg | |
| | Oral administration | | | | Intranasal administration (i.n.) |
| Number of mice | 4 | 7 | 7 | 6 | 7 |

### [Example 2]

### Confirmation of the effect of zinc and CHP on reducing allergic cytokines

After the administration of the test substances of Example 1-2, lung tissues were extracted from the mice of each group in Table 1, and RNA was extracted according to the manufacturer's total RNA isolation protocol using NucleoZOL (MACHEREY-NAGEL). 1 µg of RNA was synthesized into cDNA by reverse transcription polymerase chain reaction (RT-PCR) using iScript cDNA Synthesis Kit (Bio-Rad).

The synthesized cDNA was analyzed by performing real-time PCR using iQ SYBR Green Supermix (Bio-Rad) using primer sets of the allergic cytokines *IL-4, IL-*13, and *IL-17* genes and the mucin gene *muc5ac.* Primers for real-time PCR were synthesized by requesting Bioneer, and the specific sequences are shown in Table 2. Each gene expression value was corrected by dividing it by the expression value of the housekeeping gene *hprt.*

**[Table 2]**

| **Gene name** | **Forward primer sequence (5'→3')** | **SE Q ID NO.** | **Reverse primer sequence (5'→3')** | **SE Q ID NO.** |
|---|---|---|---|---|
| *IL-4* | TCGGCATTTTGAACGAGGTC | 1 | CGTTGCTGTGAGGACGTTTG | 2 |
| *IL-13* | CCATCTACAGGACCCAGAGG A | 3 | TTTTGGTATCGGGGAGGCTG | 4 |
| *IL-17a* | CCTCACACGAGGCACAAGT | 5 | TGAAGCTCTCCCTGGACTCA | 6 |
| *Muc5ac* | TGTGCCTGCCTGTACAATGGG | 7 | CATGTGTTGGTGCAGTCAGTAG A | 8 |
| *hprt* | AAATGTCAGTTGCTGCGTCC | 9 | TCTACCAGAGGGTAGGCTGG | 10 |

According to the experimental results, as shown in FIGS. 1 to 3, it was confirmed that Experimental Groups 1 and 2 administered a combination of Zn and CHP showed a significant reduction in the allergic cytokines IL-4, IL-13, and IL-17a, which had been increased by HDM. In particular, Experimental Group 2 showed a significant allergic cytokine reduction effect compared to not only Experimental Group 1 but also the positive control group, despite the content of Zn being significantly lowered to 1/23 to 1/24 compared to Experimental Group 1. These results indicate that the combination of zinc and CHP showed the best synergistic effect.

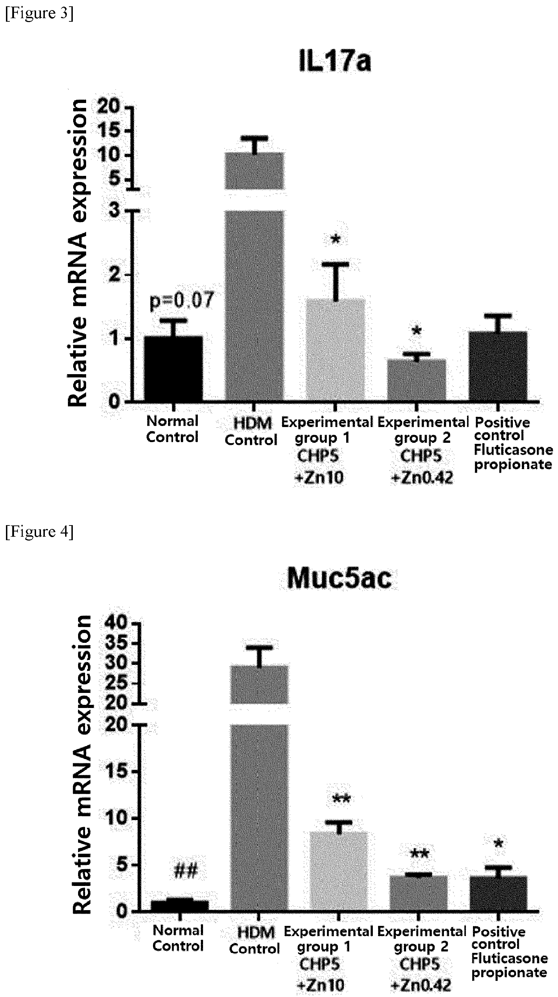

In addition, as shown in FIG. 4, the expression of the mucus gene muc5ac, which had been increased by HDM, was significantly reduced in Experimental Groups 1 and 2 administered a combination of Zn and CHP, and this effect was particularly good in Experimental Group 2.

Through this, it was confirmed that the combination of Zn and CHP exhibited an excellent effect in reducing allergic cytokines and mucus secretion, and in particular, treating zinc cations and CHP at a weight ratio of 1:11 to 13 showed the best synergistic effect, and thus is effective in treating a respiratory disease.

## Claims

1. A pharmaceutical composition for preventing or treating a respiratory disease, comprising: a zinc salt including a zinc cation and an anion; and
cyclo-hispro or a pharmaceutically acceptable salt thereof.

2. The pharmaceutical composition of claim 1, wherein the weight ratio of the zinc cation:cyclo-hispro or a pharmaceutically acceptable salt thereof is 1:11 to 13.

3. The pharmaceutical composition of claim 1, wherein the respiratory disease is one or more diseases selected from the group consisting of respiratory inflammatory lung disease, chronic obstructive pulmonary disease (COPD), sinusitis, allergic rhinitis, lower respiratory tract infections, bronchitis, emphysema, pneumonia, asthma, bronchiectasis, emphysema, sequelae of pulmonary tuberculosis, acute respiratory distress syndrome (ARDS), cystic fibrosis, otitis media, and pulmonary fibrosis.

4. The pharmaceutical composition of claim 1, wherein the bronchitis is one or more selected from the group consisting of acute bronchitis, chronic bronchitis, allergic bronchitis, infectious bronchitis, traumatic bronchitis, catarrhal bronchitis, purulent bronchitis, obstructive bronchitis, ulcerative bronchitis, and infiltrative bronchitis.

5. The pharmaceutical composition of claim 1, wherein the asthma is one or more selected from the group consisting of bronchial asthma, atopic asthma, non-atopic asthma, exercise-induced asthma, aspirin-induced asthma, psychogenic asthma, and alveolar asthma.

6. The pharmaceutical composition of claim 1, wherein the composition exhibits the effects of i) reducing allergic cytokines and ii) decreasing mucus secretion.

7. The pharmaceutical composition of claim 1, wherein the zinc salt and cyclo-hispro or a pharmaceutically acceptable salt thereof are administered simultaneously, separately, or sequentially.

8. A health functional food composition for preventing or alleviating a respiratory disease, comprising: a zinc salt including a zinc cation and an anion; and
cyclo-hispro or a sitologically acceptable salt thereof.

9. The pharmaceutical composition of claim 8, wherein the weight ratio of the zinc cation:cyclo-hispro or a sitologically acceptable salt thereof is 1:11 to 13.

10. The health functional food composition of claim 8, wherein the respiratory disease is one or more diseases selected from the group consisting of respiratory inflammatory lung disease, COPD, sinusitis, allergic rhinitis, lower respiratory tract infections, bronchitis, emphysema, pneumonia, asthma, bronchiectasis, emphysema, sequelae of pulmonary tuberculosis, ARDS, cystic fibrosis, otitis media, and pulmonary fibrosis.

11. The health functional food composition of claim 8, wherein the bronchitis is one or more selected from the group consisting of acute bronchitis, chronic bronchitis, allergic bronchitis, infectious bronchitis, traumatic bronchitis, catarrhal bronchitis, purulent bronchitis, obstructive bronchitis, ulcerative bronchitis, and infiltrative bronchitis.

12. The health functional food composition of claim 8, wherein the asthma is one or more selected from the group consisting of bronchial asthma, atopic asthma, non-atopic asthma, exercise-induced asthma, aspirin-induced asthma, psychogenic asthma, and alveolar asthma.

13. The health functional food composition of claim 8, wherein the composition exhibits the effects of i) reducing allergic cytokines and ii) decreasing mucus secretion.

14. The health functional food composition of claim 8, wherein the zinc salt and cyclo-hispro or a sitologically acceptable salt thereof are administered simultaneously, separately, or sequentially.

15. A method for preventing, alleviating, or treating a respiratory disease, comprising administering an effective amount of a composition including: a zinc salt including a zinc cation and an anion; and cyclo-hispro or a pharmaceutically acceptable salt thereof, to an individual in need thereof.

16. The method of claim 15, wherein the weight ratio of the zinc cation:cyclo-hispro or a pharmaceutically acceptable salt thereof is 1:11 to 13.

17. The method of claim 15, wherein the respiratory disease is one or more diseases selected from the group consisting of respiratory inflammatory lung disease, COPD, sinusitis, allergic rhinitis, lower respiratory tract infections, bronchitis, emphysema, pneumonia, asthma, bronchiectasis, emphysema, sequelae of pulmonary tuberculosis, ARDS, cystic fibrosis, otitis media, and pulmonary fibrosis.

18. The method of claim 15, wherein the bronchitis is one or more selected from the group consisting of acute bronchitis, chronic bronchitis, allergic bronchitis, infectious bronchitis, traumatic bronchitis, catarrhal bronchitis, purulent bronchitis, obstructive bronchitis, ulcerative bronchitis, and infiltrative bronchitis.

19. The method of claim 15, wherein the asthma is one or more selected from the group consisting of bronchial asthma, atopic asthma, non-atopic asthma, exercise-induced asthma, aspirin-induced asthma, psychogenic asthma, and alveolar asthma.

20. The method of claim 15, wherein the composition exhibits the effects of i) reducing allergic cytokines and ii) decreasing mucus secretion.

21. The method of claim 15, wherein the zinc salt and cyclo-hispro or a pharmaceutically acceptable salt thereof are administered simultaneously, separately, or sequentially.

22. A use of a composition including a zinc salt including a zinc cation and an anion; and cyclo-hispro or a pharmaceutically acceptable salt thereof, for the manufacture of a medicament for preventing, alleviating, or treating a respiratory disease.
